# EUROPEAN PATENT APPLICATION

(11) **EP 2 533 494 A1**
(43) Date of publication of application: **12.12.2012**
(21) Application number: 12171131.1
(22) Date of filing: 07.06.2012
(51) Int. Cl.: H04L 29/06, H04L 29/08

(54) **System and method of bed data aggregation, normalization and communication to third parties**

(30) Priority: 08.06.2011 US 201161494569 P
(71) Applicant: Hill-Rom Services, Inc., Batesville, IN 47006-9167 (US)
(72) Inventor: Huster, Keith, A, Sunman IN 47041 (US); Bischoff, Ryan, A, Indianapolis IN 46239 (US)
(74) Representative: Findlay, Alice Rosemary

(57) **Abstract**

A system or method of bed data aggregation, normalization, and communication to third parties is provided. A plurality of hospital beds of different bed types communicates bed data to the system and the system normalizes the data for subsequent transmission. End users select a communication protocol from among a plurality of available communication protocols in which the normalized bed data is to be transmitted to end user computer devices.

## Description

The present disclosure relates to systems and methods of handling data pertaining to hospital beds. More particularly, the present disclosure relates to a system and method of bed data aggregation, normalization and communication to third parties.

Hospital beds that send data to one or more remote computers, such one or more computers of a nurse call system, are known. In many healthcare facilities, the hospital beds and the nurse call system are made by the same manufacturer. For example, Hill-Rom Company, Inc. sells hospital beds and nurse call systems that are installed in healthcare facilities. The formatting of the data from hospital beds is typically done according to a proprietary protocol. Furthermore, there isn't any industry standard for how hospital bed data is to be formatted, how many bits or bytes of data is to be transmitted in a transmission packet, how often hospital bed data packets are to be transmitted, and so forth. As a result, the transmitted bed data formatting and content is usually different for different types of hospital beds. Thus, heretofore, third party systems (i.e., systems of entities other than the hospital bed manufacturer) such as electronic medical records (EMR) systems, admission/discharge/transfer (ADT) systems, and other systems in a healthcare facilities' information technology (IT) network were not easily able to receive and decipher hospital bed data.

A system or method of providing bed data from a plurality of hospital beds of different types to a plurality of end user computer devices has one or more of the following features alone or in any combination.

According to this disclosure, a system for use in a healthcare facility having a plurality of hospital beds of different bed types and a plurality of end user computer devices is provided. The system may include at least one server computer device having stored therein software that includes a data access software module including a plurality of interface services to interface with the plurality of hospital beds to receive bed data communicated from each hospital bed of the plurality of hospital beds. The software may have a data normalization software module to normalize the bed data into a common storage format. The software may further have a data storage software module to store the normalized bed data. Furthermore, the software may have a communication software module to communicate the normalized bed data to the plurality of end user computer devices according to a selected communication protocol that may be selected via each end user computer device of the plurality of end user computer devices from among a plurality of available communication protocols that may be provided by the communication software module.

Also according to this disclosure, the software may be programmed to permit end users to select a first time interval from among a plurality of time intervals at which the normalized data may be transmitted to the end user computer devices. The data access software module may be configured to receive the bed data from the plurality of different bed types at a second time interval that may be shorter than the first time interval.

In some embodiments, the data normalization software module may be programmed to allow an authorized end user to have access to the normalized data in a raw format. The software may be configured to receive bed commands from an authorized end user and transmit the bed commands to a designated bed of the plurality of hospital beds.

The available communication protocols may include, for example, at least two communication protocols. In some embodiments, the at least two communication protocols may include an OData Atom protocol and a JSON Endpoint protocol.

According to this disclosure, the software may be configured to permit authorized end users to associate bed location data and assigned patient data to each hospital bed of the plurality of hospital beds and to the bed data being communicated therefrom. The bed location data and the assigned patient data may be included in the normalized bed data.

In some contemplated embodiments, the communication software module may be programmed to indicate a connection state of each hospital bed of the plurality of hospital beds so that the end user computer devices are able to display which hospital beds are communicatively connected and which hospital beds are communicatively disconnected from the server computer device. The communication software module may be configured to upload software plug-ins to the plurality of end user computer devices.

According to this disclosure, a machine readable medium for receiving bed data from a plurality of hospital beds of different bed types and providing at least some of the bed data to end user computer devices may be provided. The machine readable medium may be a tangible medium, for example, that may include a plurality of instructions, that in response to being executed, may result in at least one computer device providing an interface that receives bed data from the plurality of hospital beds, normalizes the bed data into a common storage format, stores the normalized data in a database, and provides access to the normalized data to the end user computer devices according to a communication protocol selected by an end user from among a plurality of available communication protocols provided by the plurality of instructions.

The plurality of instructions may be configured to permit end users to select a first time interval from among a plurality of time intervals at which the normalized data is transmitted to the end user computer devices. The plurality of instructions, when executed, may be configured to receive the bed data from the plurality of different bed types at a second time interval that is shorter than the first time interval.

According to this disclosure, the plurality of instructions, when executed, may be configured to convert the normalized data to the communication protocol selected by the end user. In some embodiments, the plurality of instructions, when executed, may be configured to permit an authorized end user to have access to the normalized data in a raw format. Alternatively or additionally, the plurality of instructions, when executed, may be configured to receive bed commands from an authorized end user and transmit the bed commands to a designated bed of the plurality of bed types.

In some embodiments, the plurality of available communication protocols may include at least two communication protocols. For example, the at least two communication protocols may include an OData Atom protocol and a JSON Endpoint protocol. The plurality of instructions, when executed, may be configured to permit authorized end users to associate bed location data and assigned patient data to each hospital bed of the plurality of hospital beds and the bed data being communicated therefrom. The plurality of instructions, when executed, may be configured to include the bed location data and the assigned patient data in the normalized bed data.

The plurality of instructions, when executed, may provide information indicative of a connection state of each hospital bed of the plurality of hospital beds so that the end user computer devices are able to display which hospital beds are communicatively connected and which hospital beds are communicatively disconnected to at least one server computer device executing the plurality of instructions. The plurality of instructions, when executed, may upload software plug-ins to the plurality of end user computer devices.

Further according to this disclosure, a system for use with information technology (IT) infrastructure of a healthcare facility may be provided. The system may include a plurality of hospital beds of different types. The system may also include a plurality of system interfaces of different types. Each system interface of the plurality of system interfaces may be communicatively coupled to a respective hospital bed of the plurality of hospital beds and the plurality of system interfaces may be communicatively coupled to the IT infrastructure. Moreover, the system may include at least one server that may have software which includes a number of modules. For example, the software may include a data access software module that may include a plurality of interface services to interface with the plurality of system interfaces to receive bed data communicated from the plurality of hospital beds via the plurality of system interfaces, a data normalization software module to normalize the bed data into a common storage format, a data storage software module to store the normalized bed data, and a communication software module to communicate the normalized bed data to a plurality of end user computer devices according to a selected communication protocol that is selected from among a plurality of available communication protocols provided by the communication software module.

In some embodiments, at least a first group of the plurality of system interfaces may be communicatively coupled to the respective hospital beds via wired connections and at least a second group of the plurality of system interfaces may be communicatively coupled to the respective hospital beds via wireless connections. The software may be programmed to permit end users to select a first time interval from among a plurality of time intervals at which the normalized data may be transmitted to the end user computer devices. The data access software module may be configured to receive the bed data from the plurality of different bed types at a second time interval that is shorter than the first time interval.

In some embodiments, the data normalization software module may be programmed to allow an authorized end user to have access to the normalized data in a raw format. The software may be configured to receive bed commands from an authorized end user and transmit the bed commands to a designated bed of the plurality of hospital beds. As note previously, the available communication protocols may include at least two communication protocols such as an OData Atom protocol and a JSON Endpoint protocol.

The software may be configured to permit authorized end users to associate bed location data and assigned patient data to each hospital bed of the plurality of hospital beds and the bed data being communicated therefrom. The bed location data and the assigned patient data may be included in the normalized bed data, as also previously noted.

The communication software module may be programmed to indicate a connection state of each hospital bed of the plurality of hospital beds so that the end user computer devices are able to display which hospital beds are communicatively connected and which hospital beds are communicatively disconnected from the at least one server. Alternatively or additionally, the communication software module may be configured to upload software plug-ins to the plurality of end user computer devices.

Also according to this disclosure, a method of providing bed data from a plurality of hospital beds of different types to a plurality of end user computer devices may be provide. The method may comprise receiving with a server the bed data from the plurality of hospital beds. The bed data content and formatting may be different for the different types of hospital beds. The method further may include normalizing the bed data into a common storage format, storing the normalized bed date, and communicating via a communication software module the normalized bed data to the plurality of end user computer devices according to a selected communication protocol that is selected via each end user computer device of the plurality of end user computer devices from among a plurality of available communication protocols provided by the communication software module.

In some embodiments, receiving the bed data from the plurality of hospital beds may include receiving the bed data via a plurality of interface devices. Each of the interface devices may be communicatively coupled to a respective one of the plurality of hospital beds. The normalized bed data may be communicated to the plurality of end user computer devices at a user selected time interval. The user selected time interval may be longer than a time interval at which each of the plurality of beds transmits its respective bed data to the server.

The method may further comprise allowing an authorized end user to have access to the normalized data in a raw format. Furthermore, the method may also comprise receiving with the server bed commands from an authorized end user and transmitting the bed commands to a designated bed of the plurality of hospital beds. Alternatively or additionally, the method may include inputting into the server bed location data and assigned patient data to associate each hospital bed location and an assigned patient with the bed data being communicated from the respective hospital bed.

According to this disclosure, communicating via a communication software module the normalized bed data to the plurality of end user computer devices may include communicating the bed location data and the assigned patient data as part of the normalized bed data. Alternatively or additionally, the method may include uploading software plug-ins to the plurality of end user computer devices.

Further according to this disclosure, the method may permit normalized bed data to be communicated to at least one end user computer device of the plurality of end user computer devices in response to a query made in an on demand manner. A system or machine readable medium according to this disclosure may include software that may be programmed to permit end users to query for bed data in an on demand manner. Such a query may be made via a refresh button that is selected on a screen of a computer device of an end user.

The invention will now be further described by way of example with reference to the accompanying drawings, in which:

Fig. 1 is a block diagram showing a plurality of hospital beds of different bed types coupled to a bed data manager server via respective system interfaces and information technology (IT) infrastructure of a healthcare facility, the bed data manager server being coupled to a plurality of end user computer devices via the IT infrastructure, and the bed data manager server being coupled to computer devices of one or more raw data subscribers either via a dedicated connection or via the IT infrastructure;

Figs. 2A and 2B together form a block diagram, similar to Fig. 1, showing various software modules included in the bed data manager server and showing software plug-ins that are uploaded to one of the end user devices;

Fig. 3 shows an example of a screen shot of a Bed Data screen having bed data displayed on a display screen of one of the end user devices;

Fig. 4 shows an example of a screen shot of a Manage Application Settings screen including a block in which the respective end user is able to indicate a polling interval, in milliseconds (ms), at which bed data is communicated to the respective end user device;

Fig. 5 shows an example of a Detailed Bed Data screen having additional bed data displayed in a window appearing beneath a selected bed row of the Bed Data screen;

Fig. 6 shows an example of an Association screen in which an Assigned Location ID field, an Assigned Location Name field, and an Assigned Patient field are populated with information associated with each system interface listed in an Assigned Bed Connector column; and

Fig. 7 is an example of an Edit Location screen having text boxes in which the Location ID, Assigned Location Name, and Assigned Patient information can be entered for a selected system interface indicated in the BedConnector ID field.

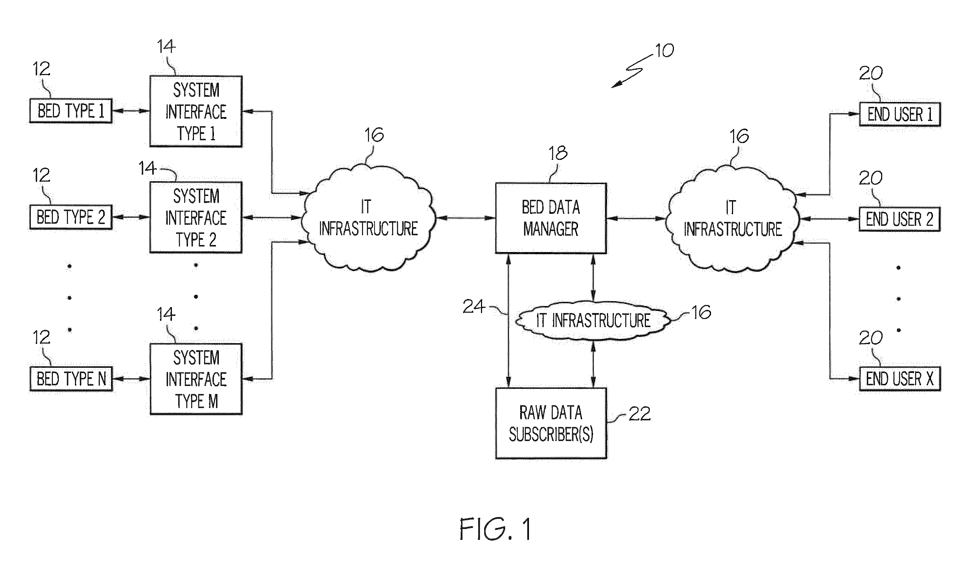

According to this disclosure a system 10 includes hospital beds 12 of different types as indicated diagrammatically in Fig. 1. Each of beds 12 communicates, either via a wired connection or a wireless connection, with a respective system interface 14. System interfaces 14 also may be of different types, although the number of types of interfaces 14 will typically not correspond to the number of bed types. Accordingly, Fig. 1 suggests that there may be "N" bed types and "M" system interface types. For example, system interfaces 14 that are able to communicate with multiple different types of hospital beds 12 are contemplated by this disclosure. Thus, N may be greater than, less than, or equal to M according to different embodiments contemplated by this disclosure. System interfaces 14 are communicatively coupled via information technology (IT) infrastructure 16 with a bed data manager 18. Bed data manager 18 is sometimes referred to herein as bed data manager server 18 or simply as server 18.

Server 18 receives bed data sent from beds 12 of the various types and normalizes the bed data into a common format for storage in a database that is either included in server 18 or associated with server 18. End user computer devices 20 access the normalized bed data via the IT infrastructure 16. According to this disclosure, server 18 has software that permits end users of computer devices 20 to select a particular communication protocol from among a plurality of available communication protocols that the normalized data is to be provided to each respective end user computer device 20. As suggested in Fig. 1, there may be up to "X" end user computer devices 20 that receive normalized bed data according to a selected communication protocol from server 18. The number X of end user computer devices 20 may be greater than, less than, or equal to M and/or N according to different embodiments contemplated by this disclosure. In some embodiments, server 18 is configured to receive bed data from up to five hundred (500) beds 12.

As also shown diagrammatically in Fig. 1, server 18 is configured to transmit bed data to computer devices 22 in a raw data format. That is, the bed data is transmitted to computer devices 22 in the same format as it is received from the various beds 12. Server 18 communicates with one or more of computer devices 22 via IT infrastructure 16 in some embodiments. Alternatively or additionally, server 18 communicates with one or more computer devices 22 via a dedicated communication link 24 which may be a wired datalink or wireless datalink according to this disclosure. Just to be clear, in some embodiments, one group of computer devices 22 may communicate with server 18 via IT infrastructure 16 and another group of computer devices 22 may communicate with server 18 via respective dedicated communication links 24. Similarly, some or all of end user computer devices 20 may communicate with server 18 via dedicated communication links (not shown) in lieu of IT infrastructure 16. The computer devices 20, 22 communicating via dedicated communication links are co-located with server 18, in some instances, thereby enabling a direct wired connection to server 18.

IT infrastructure 16, as illustrated diagrammatically in Fig. 1, is intended to encompass the various other hardware and software that exists in a facility, such as a healthcare facility, or across multiple facilities, that permits computer devices, such as beds 12, system interfaces 14, computer devices 20, 22, and so forth to communicate with at least one other computer device. IT infrastructure typically includes things such as gateways, routers, servers, transmitters, receivers, transceivers, wiring, connection ports, and so on. The hardware and software of the IT infrastructure is typically acquired and installed in a facility at a time different than the time at which a facility may acquire and install beds 12, interfaces 14, server 18, and computer devices 20, 22.

In some embodiments, server 18 transmits raw bed data to computer devices 22 at the same frequency with which it is received from the various beds 12. Thus, if beds 12 are transmitting bed data at a time interval of, say, every 100 milliseconds, then server 18, in turn, transmits the bed data to computer devices 22 about every 100 milliseconds. Accordingly, it is contemplated that users who wish to receive bed data in its raw format at such high frequency must subscribe to server 18 for this purpose. In contrast and as will be discussed in further detail below, users of computer devices 20 typically will not wish to receive bed data as frequently as it is received by server 18. According to this disclosure, therefore, users of computer devices 20 are able to select the frequency at which server 18 transmits bed data to their respective computer devices 20. The time interval or frequency at which server 18 transmits bed data to computers 20 can vary from computer 20 to computer 20 according to this disclosure.

System interfaces 14 may sometimes include bed interface units (BIU's), network interface units (NIU's), or wireless interface units (WIU's) of the type available from Hill-Rom Company, Inc. Discussion of the functionality and details of BIU's, NIU's and WIU's may be found in U.S. Patent Nos. 7,868,740; 7,852,208; 7,746,218; 7,538,659 and 7,319,386 and in U.S. Patent Application Publication Nos. 2009/0217080; 2009/0214009; 2009/0212956; and 2009/0212925, for example. Other types of system interfaces 14 include Ethernet ports such as RJ-45 connector ports as well as RS-232 ports or the like. Wireless access points (WAP's) are also considered to be system interfaces 14 according to this disclosure.

Hospital beds 12 are of different types as previously mentioned. The term hospital bed as used herein is intended to cover beds of all types and in all environments, not just those found in hospitals. Of course, the beds 12 contemplated herein have the ability to communicate bed data to remote computer devices. In some instances, the beds 12 are also able to receive data or commands from remote computer devices. It will be appreciated that each different type of bed 12 typically will communicate different types of data and the data may be formatted in different ways. Table 1 below is an example of the type of bed data that is available from six different types of hospital beds 12.

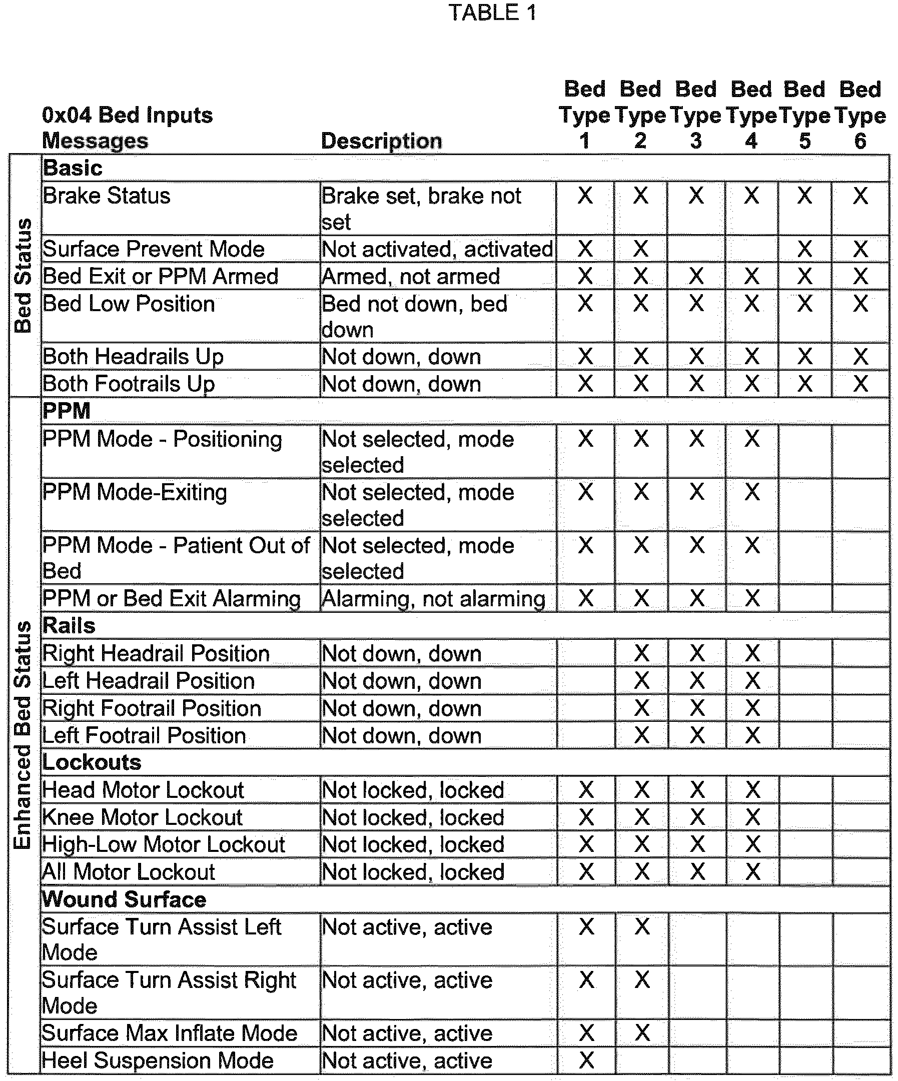

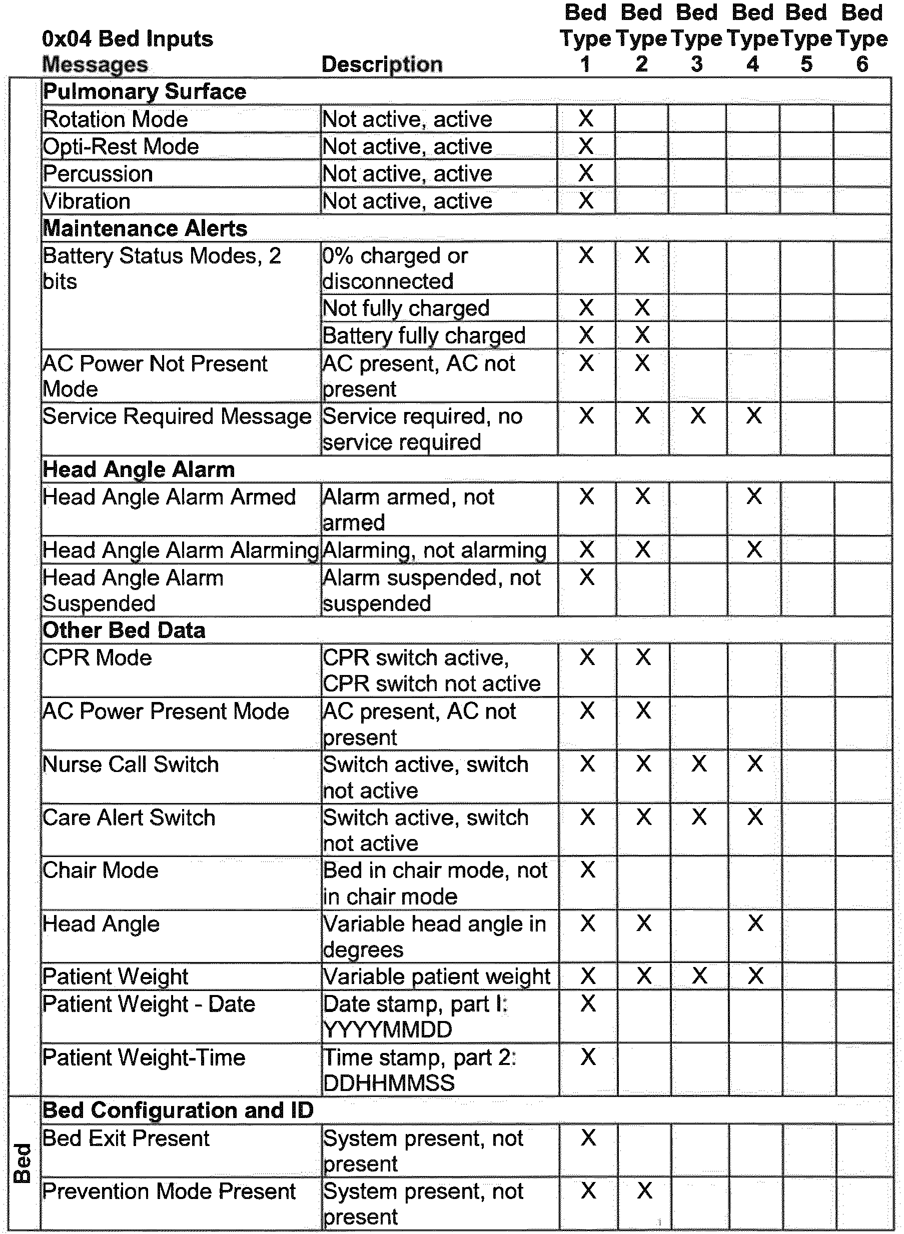

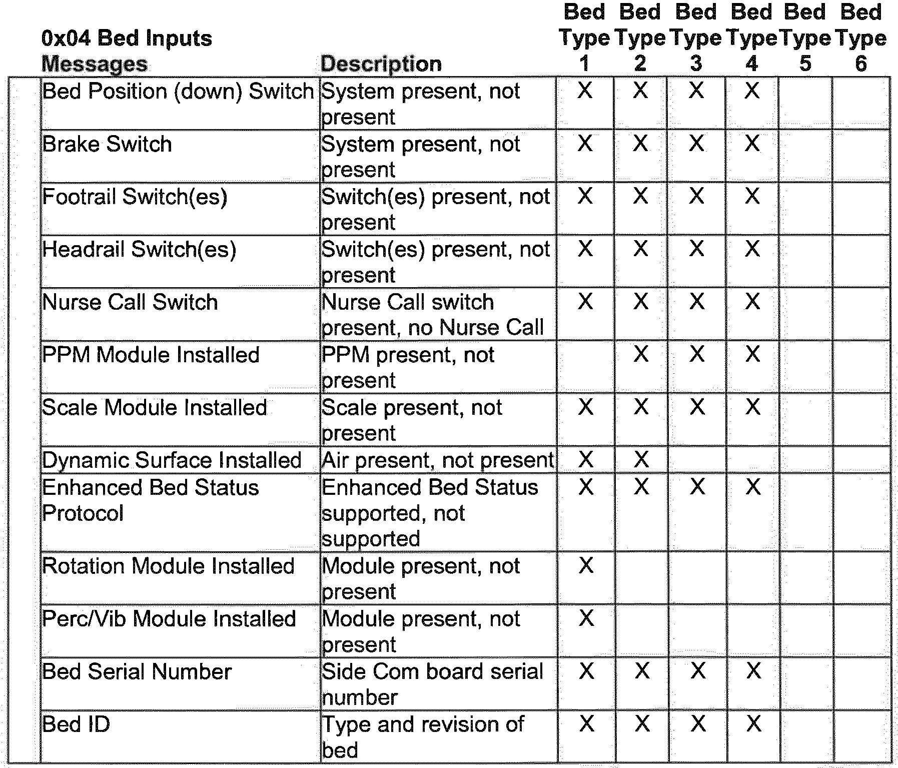

In the example of Table 1, Bed Type 1 is the TOTALCARE® bed, Bed Type 2 is the VERSACARE® bed, Bed Type 3 is the CAREASSIST® ES bed, Bed Type 4 is the ADVANTA™ 2 bed, Bed Type 5 is the ADVANCE™ bed, and Bed Type 6 is the ADVANTA™ bed, each of which is or was marketed by Hill-Rom Company, Inc. Beds 12 of other types which have other types of bed data are, of course, within scope of this disclosure. Based on Table 1, it will be appreciated that the number of bits being transmitted from beds 12 of different types may vary widely depending upon the amount of information to be conveyed. According to one embodiment of system 10 contemplated by this disclosure, server 18 is capable of receiving data from up to five hundred (500) beds 12.

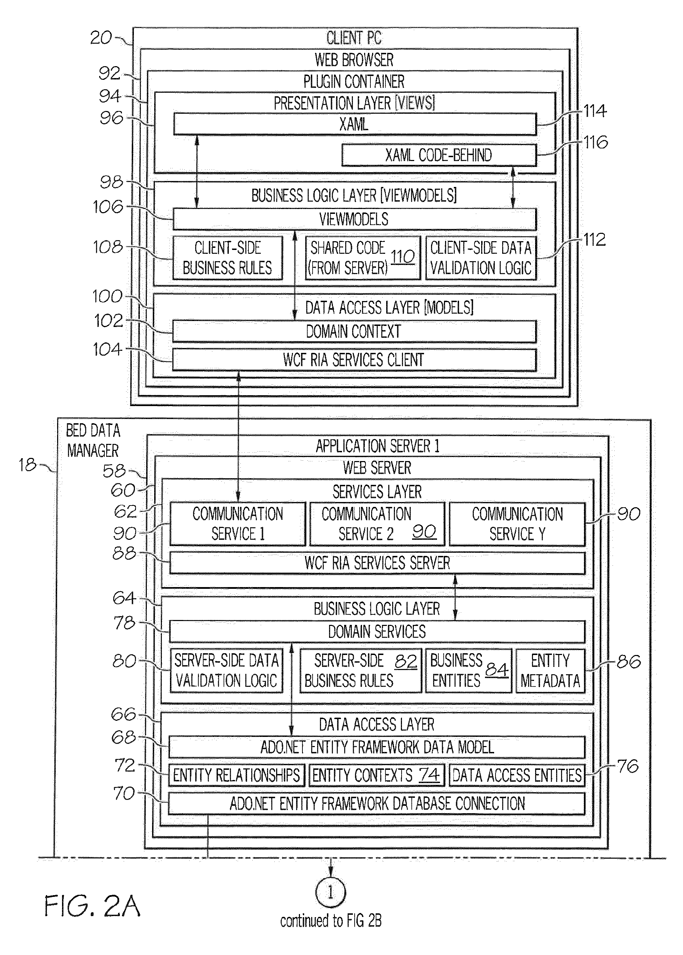

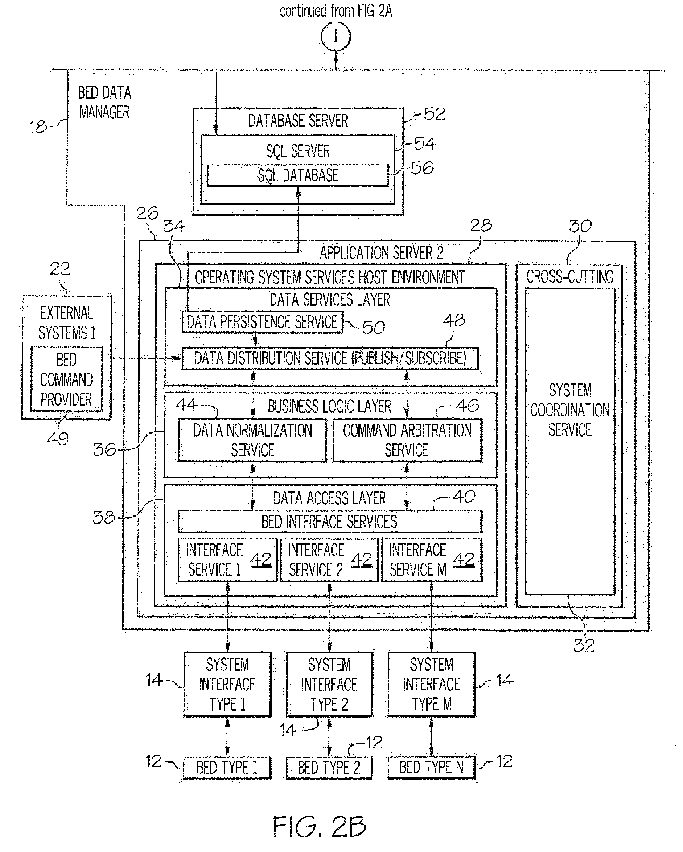

Referring now to Figs. 2A and 2B, various software modules are shown diagrammatically as being included in bed data manager server 18. The various modules comprise software code having the associated functions as herein described. In some embodiments, the modules may be included in separate software packages that are installed in the memory of server 18 via copying from associated disks or uploaded separately to server 18 from one or more other computer devices. In other embodiments, the modules are included in a single software package or software routine such that each module is a sub-portion or subroutine of the overall routine. Regardless of whether the modules described below are provided to server 18 in a piecemeal fashion or as a single, large software routine, the modules work together to perform the functions described herein. Server 18 executes the instructions included in the software modules stored in memory of server 18.

As shown in Fig. 2B, the software stored in memory of server 18 includes an Application Server 2 module 26 which, in turn, includes an Operating System Services Host Environment module 28 and a Cross-Cutting module 30. Cross-Cutting Module 30 includes a System Coordination Service module 32. Operating System Services Host Environment module 28 includes a Data Services Layer module 34, a Business Logic Layer module 36 and a Data Access Layer module 38. The Data Access Layer module 38 includes a Bed Interface Services module 40 which includes Interface Service 1 through M modules 42. Modules 42 include the software that allows server 18 to receive bed data from the various system interfaces 14. In Figs. 2A and 2B, the IT infrastructure 16 is omitted.

Business Logic Layer module 36 includes a Data Normalization Service module 44 and a Command Arbitration Service module 46. The Data Normalization Service module 44 includes the software instructions that, when executed, normalizes the incoming bed data from the various bed types 12 into a common format. Such software instructions for normalizing the bed data, therefore, rearranges and modifies the incoming bits and bytes of data into a single, common format. The Command Arbitration Service module 46 includes software instructions that when executed, passes the bed data in its raw format (i.e., the format in which it is received from each bed 12) to a Data Distribution Service (Publish/Subscribe) module 48 of a Data Services Layer module 34 which, in turn, publishes or sends the raw bed data to one or more computer devices 22 that have subscribed to receive such raw bed data.

Command Arbitration Service module 46 also includes software instructions that, when executed, passes bed command messages received from computer devices 22 to the bed 12 for which the bed command message is designated. Module 46 sends each bed command message to module 40 which, in turn, sends the bed command message through the appropriate module 42 and system interface 14 to the appropriate bed 12. As indicated diagrammatically in Fig. 2B, the illustrative computer device 22 includes a Bed Command Provider module 49 which includes the software that, when executed, sends the bed command messages to module 48 of server 18.

In some embodiments, the programming of module 49 is done by the same party that manufactures one or more of the various bed types 12 and is installed specifically on computer device 22 as its own software package or as part of a larger software package. In other embodiments, module 49 is uploaded to computer device 22 from server 18. An example of a bed command that is sent from computer devices 22 to beds 12 include commands to arm or disarm a patient position monitoring system of the bed 12. Computer devices 22 that do not contain module 49 are within in the scope of this disclosure. That is, systems 10 in which some, all, or none of computer devices 22 include module 49 are contemplated by this disclosure.

Data Services Layer module 34 also includes a Data Persistence Service module 50. Server 18 includes a Database Server module 52 which includes, for example, an SQL Server module 54 which has an SQL database 56. Data Persistence Service module 50 passes normalized bed data to the SQL database 56 for storage. In some embodiments, as bed data from a particular bed 12 changes, the SQL database 56 is updated only with the changing data and the other, unchanged data remains. In other embodiments, the SQL database 56 is updated with all of the bed data received from each of the beds 12 regardless of whether some of the data has not changed from the prior data transmission from a particular bed.

Server 18 also includes an Application Server 1 module 58 which includes a Web Server module 60 as shown in Fig. 2A. Module 60, in turn, includes a Services Layer module 62, a Business Logic Layer module 64 and a Data Access Layer module 66. Module 66 includes an ADO.Net Entity Framework Data Model module 68 which further includes an ADO.Net Entity Framework Database Connection module 70 that interfaces with SQL Server module 54 to obtain normalized bed data. Module 66 also includes an Entity Relationships module 72 to maintain entity relationships, an Entity Contexts module 74 to maintain entity contexts, and a Data Access Entities module 76.

Business Logic Layer module 64 includes a Domain Services module 78, a Server-Side Data Validation Logic module 80, a Server-Side Business Rules module 82, a Business Entities module 84, and an Entity Metadata module 86. The Domain Services module 78 interfaces with the ADO.Net Entity Framework Data Model module 68 as shown in Fig. 2A. Services Layer module 62 include a WCF RIA Services Server module 88 and Communication Service 1 through Y modules 90. Module 88 interfaces with module 78. Modules 90 communicate with each computer device 20 that desires to receive normalized bed data according to a respective data communication protocol associated with each of Communication Service 1 through Y modules 90. According to this disclosure, the number "X" of computer devices 20 in system 10 may be greater than, equal to, or less than the number "Y" of Communication Service modules 90.

In the illustrative example, Communication Service 1 module 90 communicates with computer 20. It should be understood, based on this disclosure, that each of modules 90 may be in communication with a multitude of computer devices 20 even though Fig. 2A illustrates only one module 90 communicating with one computer device 20. In some embodiments, modules 90 of server 18 include two modules 90 that communicate normalized bed data according to two different communication protocols. In some such embodiments, the communication protocols include an OData Atom protocol and a JSON Endpoint protocol. Computer devices 20 may be included as part of an electronic medical records (EMR) system, an admission/discharge/transfer (ADT) system, a workflow system, a nurse call system, or any other computer system for which there is a reason to consume, have access to, or store bed data from beds 12.

As also shown in Fig. 2A computer device 20 includes a Web Browser module 92 with a Plugin Container module 94. According to this disclosure, modules 90 of server 18 upload software plug-ins to each Plugin Container module 94 of computer devices 20. In the illustrative example, the software plug-ins uploaded via the associated Communication Service 1 module 90 includes a Presentation Layer [Views] module 96, a Business Logic Layer [Viewmodels] module 98, and a Data Access Layer [Models] module 100. Module 100 includes a Domain Context module 102 and a WCF RIA Services Client module 104 which, in the illustrative example, is communicatively coupled to Communication Service 1 module 90. Module 98 includes a Viewmodels module 106, a Client-Side Business Rules module 108, a Shared Code (from Server) module 110, and a Client-Side Data Validation Logic module 112. Module 106 and module 102 exchange data as indicated in Fig. 2A.

Module 96 includes an XAML module 114 and an XAML Code-Behind module 116. Modules 114, 116 each exchange data with module 106. Modules 96, 98, 100, 102, 104, 106, 108, 110, 112, 114, 116 cooperate to access and display bed data on the display screen of computer device 20. As indicated above, computer device 20 is used to select the communication protocol which is desired from among the various available communication protocols associated with Communication Service 1 through Y modules 90.

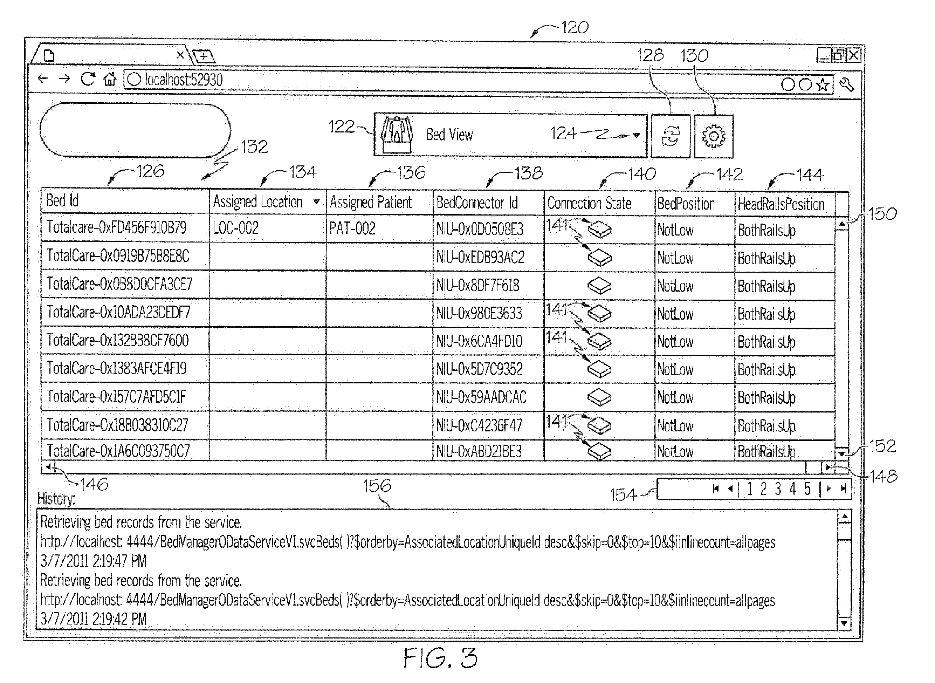

Referring now to Fig. 3, an example of a screen shot of a Bed Data screen 120 having bed data displayed on a display screen of one of the end user devices 20 is shown. Screen 120 includes a view selector box 122 having an arrow 124 that can be selected for a drop down menu of additional types of views. In the example of Fig. 3, a "Bed View" is shown which has bed data shown in table 126. A refresh button 128 is provided on screen 120 next to box 124. Selection of button 128 results in the data of table 126 being updated with the most current data. Screen 120 also has a manage settings button 130 that is selected to customize the type of data to be displayed in table 126. It will be appreciated that any of the various types of data listed above in Table 1 is selectable for inclusion in table 126 of screen 120.

In the illustrative example of Fig. 3, table 126 includes a Bed ID column 132 in which the bed type is indicated in text (e.g., "Totalcare") and in which the assigned bed identification number for the particular bed is indicated after a hyphen, an Assigned Location column 134 which indicates the bed's location in the healthcare facility, an Assigned Patient column 136 which indicates the patient assigned to the particular bed, a Bed Connector ID column 138 in which the type of system interface 14 is indicated in text (e.g., NIU) and in which the associated identification number for the system interface 14 is indicated after a hyphen, a Connection State column 140 which includes icons 141 that indicate whether or not each bed is properly connected to server 18 via interface 14, a Bed Position column 142 which indicates whether or not an upper frame of each bed 12 is in its lowest position, and a Head Rails Position column 144 which has text indicative of the position of the siderails of the associated bed 12. Left and right scroll arrows 146, 148 are provided to scroll on table 126 to see additional columns that are hidden from view. Up and down scroll arrows 150, 152 are provided to scroll up and down table 126 to see additional rows that are hidden from view. Alternatively, a paging control box 154 is provided with numbers (e.g., 1-5) which are selectable to jump to additional pages of table 126 and with arrows that are selectable to go forward or back by one page or to jump to the last page or first page of table 126. Finally, screen 120 includes a history window 156 beneath table 126 which shows the date and time at which table 126 was populated with bed data.

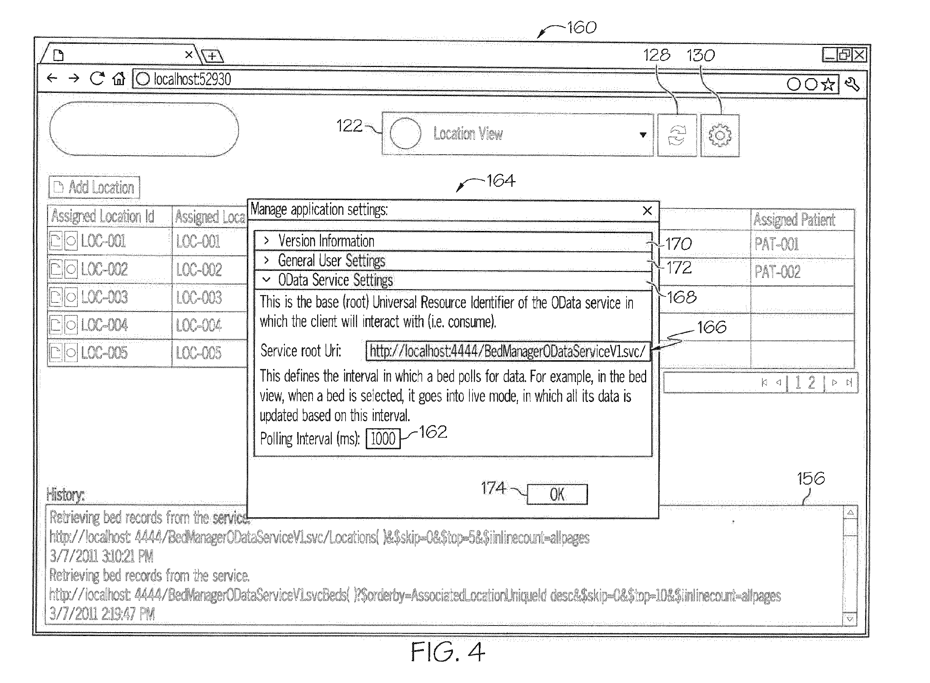

In the illustrative example of window 156 of screen 120, it is indicated that bed data was retrieved from server 18 by the computer device 20 displaying screen 120 on 3/7/2011 at 2:19:42 PM and then again on 3/7/2011 at 2:19:47 PM. Thus, in the illustrative example, computer device 20 is configured to receive and/or retrieve the bed data every five (5) seconds. According to this disclosure, the frequency at which each computer device is able to retrieve or receive bed data is selectable. When a user is first setting up and configuring the software of an associated computer 20, after a successful initial login, a Manage Application Settings screen 160 appears on the display of computer device 20 as shown, for example, in Fig. 4.

A text block 162 is provided in a Manage application settings window 164 of screen 160. The end user types in block 162 a polling interval, in milliseconds (ms), at which bed data is communicated to or retrieved by the respective end user device 20. In the illustrative example of Fig. 4, one thousand (1,000) ms has been entered in box 162. Window 164 further includes a Service root URI text block 166 in which the Universal Resource Identifier (URI) of server 18 with which the associated computer device 20 will interact is indicated. If server 18 comprises multiple server devices (e.g., the Application Server 1 module 58 is on one server, the Database Server module 52 is on another server, and the Application Server 2 module 26 is on yet another server, with the three servers communicatively coupled), then the URI entered into block 166 should be the server that includes the selected Communication Service 1 through Y module 90. In block 166, the text string preceding the ".svc" is what determines which of Communication Service 1 through Y modules 90 has been selected by the end user.

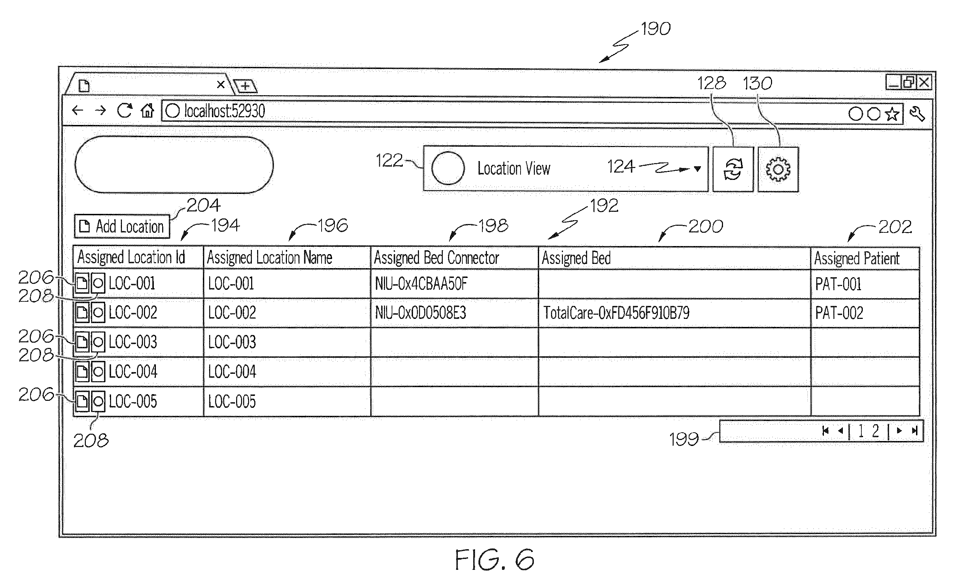

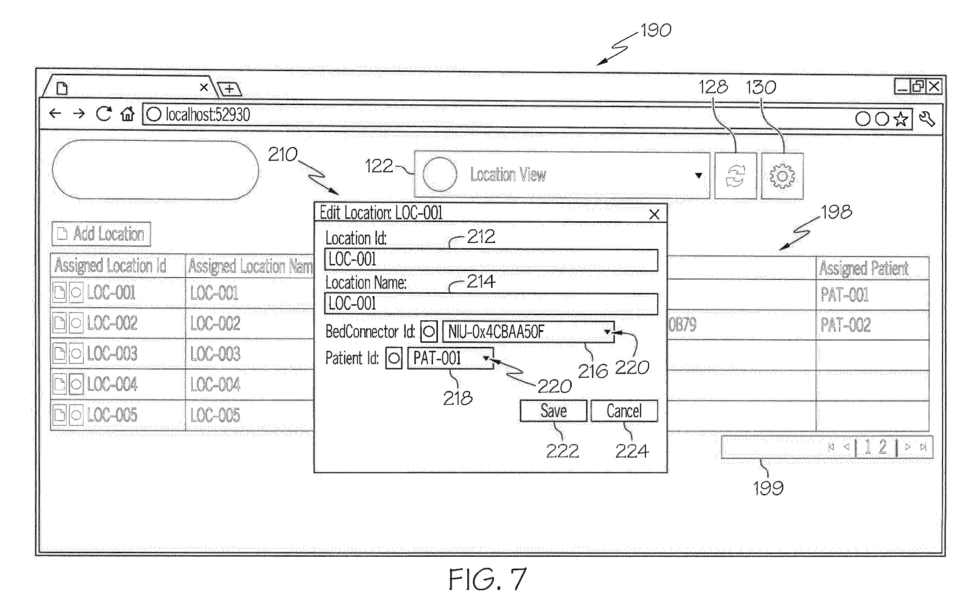

In window 164, text boxes 162, 166 appear when a user selects a Service Settings menu bar 168. Window 164 also includes a Version Information window bar 170 and a General User Settings menu bar 172. If the user selects bar 170, information about the software version uploaded to the user's computer device 20 in modules 96, 98, 100, for example, is shown. If the user selects bar 172, the user is able to toggle history window 156 on and off via selection of check boxes or radio buttons or the like that appear in an associated drop down window. After the user finishes making the selections and entering the relevant information in boxes 162, 166, the user selects an OK button 174 to close window 164. In the illustrative example of Fig. 4, the text "Location View" appears in the view selector box 122. Details of the Location View are discussed below in connection with Figs. 6 and 7.

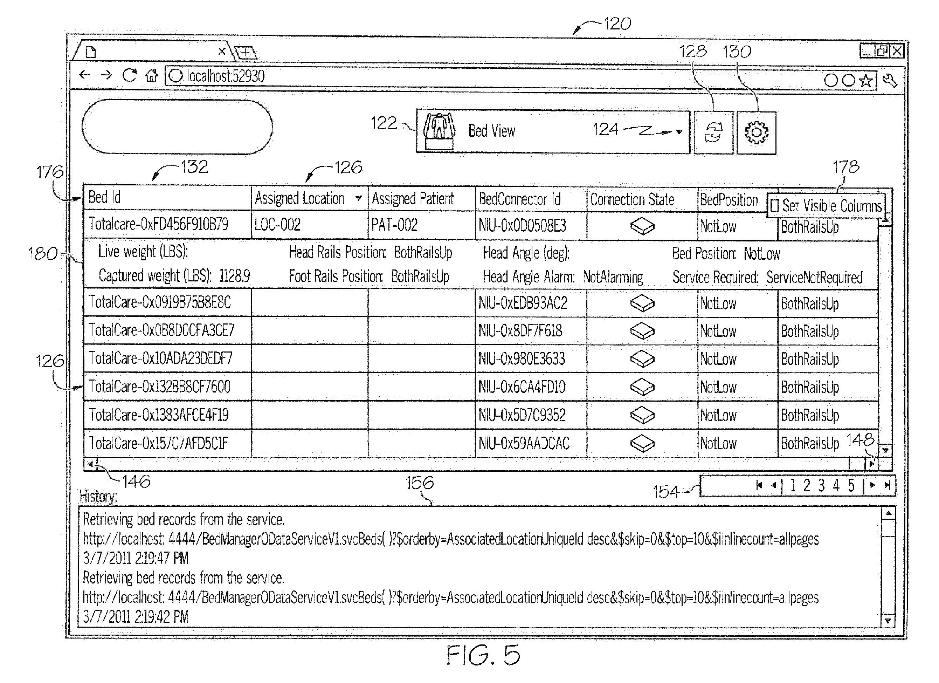

Referring now to Fig. 5, a screen which is similar to Fig. 3, but which illustrates a couple additional features available on the Bed Data screen 120, is shown. One additional feature allows a user to right click on the table header row 176 which results in a bed column chooser box 178 to appear on table 126. The term "right click" means that a user has placed a cursor over row 176 using a computer mouse and then clicked on a mouse button that is on the right hand side of the mouse as is generally understood by those familiar with a computer mouse. By selecting the bed column chooser box 178, a pop up window (not shown) appears with the list of all of the available bed column names that may be displayed in the bed view. The user then selects or deselects the various bed columns names using a check box or radio button or the like. Based on the description above, it will be understood that if a user selects more columns than can be seen in table 126 at the same time, then the left and right scroll arrows 146, 148 appear to permit a user to scroll to the unseen columns as desired.

Another feature of the Bed Data screen 120 shown in Fig. 5 is a polling window 180. Polling window 180 appears in table 126 when a user selects a particular row of bed data on table 126 via a left mouse click, for example, or by using up and down arrow keys of a tab key to highlight a particular row of bed data then hitting the enter key of a keyboard of the respective computer device 20. When the polling window 180 appears, the selected row is now live in the sense that it will get bed data updates in near real-time. That is, the data for the particular bed will be updated at the frequency that the data is transmitted from the respective bed 12 rather than being updated at the frequency selected using box 162 on screen 160. By "near real-time" it is intended to mean in approximately real-time taking into account the data processing delays that inherently exist as server 18 and computer 20 process the bed data through the various software modules. This is a practical feature when monitoring a single bed 12 for data updates because it eliminates the need to continuously or repeatedly select the refresh button 128.

Referring now to Fig. 6, when a user selects the Location View from the drop down menu that appears near box 122 in response to icon 124 being selected, an Association screen 190 appears on the display screen of the respective computer device 20. Association screen 190 includes an Association table 192 which, in turn, includes an Assigned Location ID column 194, an Assigned Location Name column 196, an Assigned Bed Connector column 198, an Assigned Bed column 200, and an Assigned Patient column 202. According to this disclosure, an authorized user of computer device 20 is able to add, update, and delete data appearing in table 192. Thus, table 192 is used to create the associations between location ID's, location names, the ID's of system interfaces 14, the ID's of beds 12, and the patients assigned to beds 12. If table 192 has more rows of information than can be seen at one time, a paging control box 199 is provided to navigate to the additional rows of information by selecting the forward or back arrows or by selecting the page number appearing in box 199.

In the illustrative example of table 192 in Fig. 6, the location names appearing in column 196 match the location ID's appearing in column 194. However, this need not be the case such that a particular healthcare facility may have location names that are different than the location ID's. Also, the manner in which the assigned patient is indicated in column 202 may be in any number of formats not just one the shown. Thus, rather than PAT-001 for the assigned patient, a common alternative is "Sm... Jo" which has the first two letters of the patient's last name and first two letters of the patient's first name separated by ellipses. Of course, some other manner of indicating an assigned patient, such as a patient's full name, could be used, if desired, in column 202.

Screen 190 includes an Add Location button 204 that is selected to add a new location to the list in table 192. Screen 190 also includes an edit icon or button 206 and a delete icon or button 208 in each row of column 194. If delete button 208 is selected, the information appearing in the associated row of table 192 is deleted. If button 204 or button 206 is selected, then a pop-up window 210 appears on screen 190 as shown, for example, in Fig. 7. However, if button 204 is selected to add a location to table 192, then the various text boxes of window 210 are blank, whereas if button 206 is selected to edit the information of a location already appearing in table 192, then the various text boxes are filled with the current data from table 192 from the associated row of table 190 for which icon 206 was selected.

Window 210 includes a Location ID text box 212, a Location Name text box 214, a Bed Connector ID text box 216 and a Patient ID text box 218 as shown in Fig. 7. The user of computer device 20 types into boxes 212, 214 whatever location ID and location name, respectively, the user wishes to type in those boxes. It will be appreciated that different healthcare facilities will have their own conventions for designating location ID's and location names. Boxes 216, 218 each have a respective menu drop down arrow 220 that can be selected to retrieve a list of the Bed Connector ID's (e.g., the type and serial number of the system interfaces 14 that are in communication with computer device 20 via server 18) and a list of patient's that are available for associating with the room location. Selection of one the Bed Connector ID's corresponding to one of the system interfaces 14 in window 210 will automatically cause the associated bed ID to appear in column 200 of table 192 and in column 132 of table 126 since each system interface 14 is coupled to a corresponding bed 12.

Window 210 has a Save button 222 and a Cancel button 224 as shown in Fig. 7. After the user of computer device 20 has filled in boxes 212, 214, 216, 218 with the desired information, the user selects button 222 to save the entered information. If the user had selected Add Location button 204 then the saved information appears as a new row in table 198, otherwise if the user was editing an existing row of table 198, then the saved information appears in the edited row. Alternatively, if the user does not wish to save the information entered into boxes 212, 214, 216, 218, the user selects button 224 and the user will close out of window 210 without the information in boxes 212, 214, 216, 218 being added or modified on table 198.

Although certain embodiments have been described in detail above, variations and modifications exist.

## Claims

1. A system for use in a healthcare facility having a plurality of hospital beds of different bed types and a plurality of end user computer devices, the system comprising at least one server computer device having stored therein software that includes a data access software module including a plurality of interface services to interface with the plurality of hospital beds to receive bed data communicated from each hospital bed of the plurality of hospital beds, a data normalization software module to normalize the bed data into a common storage format, a data storage software module to store the normalized bed data, and a communication software module to communicate the normalized bed data to the plurality of end user computer devices according to a selected communication protocol that is selected via each end user computer device of the plurality of end user computer devices from among a plurality of available communication protocols provided by the communication software module.

2. The system of claim 1, wherein the software is programmed to permit end users to select a first time interval from among a plurality of time intervals at which the normalized data is transmitted to the end user computer devices.

3. The system of claim 2, wherein the data access software module is configured to receive the bed data from the plurality of different bed types at a second time interval that is shorter than the first time interval.

4. The system of any preceding claim, wherein the data normalization software module is programmed to allow an authorized end user to have access to the normalized data in a raw format.

5. The system of any preceding claim, wherein the software is configured to receive bed commands from an authorized end user and transmit the bed commands to a designated bed of the plurality of hospital beds.

6. The system of any preceding claim, wherein the available communication protocols comprises at least two communication protocols.

7. The system of claim 6, wherein the at least three communication protocols include an OData Atom format and a JSON Endpoint format.

8. The system of any preceding claim, wherein the software is configured to permit authorized end users to associate bed location data and assigned patient data to each hospital bed of the plurality of hospital beds and the bed data being communicated therefrom.

9. The system of claim 8, wherein the bed location data and the assigned patient data is included in the normalized bed data.

10. The system of any preceding claim, wherein the communication software module is programmed to indicate a connection state of each hospital bed of the plurality of hospital beds so that the end user computer devices are able to display which hospital beds are communicatively connected and which hospital beds are communicatively disconnected from the server computer device.

11. The system of any preceding claim, wherein the communication software module is configured to upload software plug-ins to the plurality of end user computer devices.

12. The system of any preceding claim, wherein the software is programmed to permit end users to query for bed data in an on demand manner.

13. The system of any preceding claim further comprising a plurality of hospital beds of different types, a plurality of system interfaces of different types, each system interface of the plurality of system interfaces being communicatively coupled to a respective hospital bed of the plurality of hospital beds, the plurality of system interfaces being communicatively coupled to information technology (IT) infrastructure of the healthcare facility.

14. A machine readable medium for receiving bed data from a plurality of hospital beds of different bed types and providing at least some of the bed data to end user computer devices, the machine readable medium being a tangible medium and comprising a plurality of instructions, that in response to being executed, results in at least one computer device providing an interface that receives bed data from the plurality of hospital beds, normalizes the bed data into a common storage format, stores the normalized data in a database, and provides access to the normalized data to the end user computer devices according to a communication protocol selected by an end user from among a plurality of available communication protocols provided by the plurality of instructions.

15. A method of providing bed data from a plurality of hospital beds of different types to a plurality of end user computer devices, the method comprising
receiving with a server the bed data from the plurality of hospital beds, wherein the bed data content and formatting is different for the different types of hospital beds,
normalizing the bed data into a common storage format,
storing the normalized bed date, and
communicating via a communication software module the normalized bed data to the plurality of end user computer devices according to a selected communication protocol that is selected via each end user computer device of the plurality of end user computer devices from among a plurality of available communication protocols provided by the communication software module.
